# EUROPEAN PATENT APPLICATION

(11) **EP 3 278 816 A1**
(43) Date of publication of application: **07.02.2018**
(21) Application number: 17184791.6
(22) Date of filing: 03.08.2017
(51) Int. Cl.: A61K 48/00, C12Q 1/68

(54) **MODIFIED CANCER CELL LINES AND USES THEREOF**

(30) Priority: 04.08.2016 US 201662371091 P
(71) Applicant: Chang Gung Memorial Hospital, Linkou, Taoyuan City 33305 (TW)
(72) Inventor: YU, Alice L., 33305 Taoyuan City (TW); LIN, Wen-Der, 33305 Taoyuan City (TW); HUNG, Jung-Tung, 33305 Taoyuan City (TW); FAN, Tan-chi, Taoyuan City (TW)
(74) Representative: Cabinet Becker et Associés

(57) **Abstract**

The present invention relates to modified cancer cell lines, wherein the ST3Gal1 glycosyltransferase expression is reduced or inhibited. The use of the modified cancer cell lines with suppressed ST3Gal1 glycosyltransferase to screen a potential cancer therapeutic is provided. The suppression of ST3Gal1 glycosyltransferase expression, with or without an anticancer agent is also provided to inhibit the growth of cancer cells in a subject.

SEQUENCE LISTING

<110>  Chang Gung Memorial Hospital

<120>  MODIFIED CANCER CELL LINES AND THE USES THEREOF

<130>  B2546EP

<160>  15

<170>  PatentIn version 3.5

<210>  1
<211>  1023
<212>  DNA
<213>  Homo sapiens

<400>  1
atggtgaccc tgcggaagag gaccctgaaa gtgctcacct tcctcgtgct cttcatcttc      60

ctcacctcct tcttcctgaa ctactcccac accatggtgg ccaccacctg gttccccaag     120

cagatggtcc tggagctctc cgagaacctg aagagactga tcaagcacag gccttgcacc     180

tgcacccact gcatcgggca gcgcaagctc tcggcctggt tcgatgagag gttcaaccag     240

accatgcagc cgctgctgac cgcccagaac gcgctcttgg aggacgacac ctaccgatgg     300

tggctgaggc tccagcggga gaagaagccc aataacttga atgacaccat caaggagctg     360

ttcagagtgg tgcctgggaa tgtggaccct atgctggaga agaggtcggt gggctgccgg     420

cgctgcgccg ttgtgggcaa ctcgggcaac ctgagggagt cttcttatgg gcctgagata     480

gacagtcacg actttgtcct caggatgaac aaggcgccca cggcagggtt tgaagctgat     540

gttgggacca agaccaccca ccatctggtg taccctgaga gcttccggga gctgggagat     600

aatgtcagca tgatcctggt gcccttcaag accatcgact tggagtgggt ggtgagcgcc     660

atcaccacgg gcaccatttc ccacacctac atcccggttc ctgcaaagat cagagtgaaa     720

caggataaga tcctgatcta ccacccagcc ttcatcaagt atgtctttga caactggctg     780

caagggcacg ggcgataccc atctaccggc atcctctcgg tcatcttctc aatgcatgtc     840

tgcgatgagg tggacttgta cggcttcggg gcagacagca aagggaactg gcaccactac     900

tgggagaaca acccatccgc ggggggcttt cgcaagacgg gggtgcacga tgcagacttt     960

gagtctaacg tgacggccac cttggcctcc atcaataaaa tccggatctt caaggggaga    1020

tga                                                                  1023


<210>  2
<211>  25
<212>  RNA
<213>  Artificial Sequence

<220>
<223>  siRNA oligonucleotides against ST3Gal1, antisense strand

<400>  2
ucacucugau cuuugcagga accgg                                          25


<210>  3
<211>  25
<212>  RNA
<213>  Artificial Sequence

<220>
<223>  siRNA oligonucleotides against ST3Gal1, sense strand

<400>  3
ccgguuccug caaagaucag aguga                                          25


<210>  4
<211>  25
<212>  RNA
<213>  Artificial Sequence

<220>
<223>  siRNA oligonucleotides against ST3Gal1, antisense strand

<400>  4
aucucaggcc cauaagaaga cuccc                                          25


<210>  5
<211>  25
<212>  RNA
<213>  Artificial Sequence

<220>
<223>  siRNA oligonucleotides against ST3Gal1, sense strand

<400>  5
gggagucuuc uuaugggccu gagau                                          25


<210>  6
<211>  25
<212>  RNA
<213>  Artificial Sequence

<220>
<223>  siRNA oligonucleotides against ST3Gal1, antisense strand

<400>  6
ucaugcugac auuaucuccc agcuc                                          25


<210>  7
<211>  25
<212>  RNA
<213>  Artificial Sequence

<220>
<223>  siRNA oligonucleotides against ST3Gal1, sense strand

<400>  7
gagcugggag auaaugucag cauga                                          25

<210>  8
<211>  25
<212>  RNA
<213>  Artificial Sequence

<220>
<223>  siRNA oligonucleotides against ST3Gal1, antisense strand

<400>  8
uugagaagau gaccgagagg augcc                                          25


<210>  9
<211>  25
<212>  RNA
<213>  Artificial Sequence

<220>
<223>  siRNA oligonucleotides against ST3Gal1; sense strand

<400>  9
ggcauccucu cggucaucuu cucaa                                          25


<210>  10
<211>  58
<212>  DNA
<213>  Artificial Sequence

<220>
<223>  ST3Gal1 shRNA, oligo sequence

<400>  10
ccggagagac ttgagtggcg attacctcga ggtaatcgcc actcaagtct ctttttg       58


<210>  11
<211>  58
<212>  DNA
<213>  Artificial Sequence

<220>
<223>  ST3Gal1 shRNA, oligo sequence

<400>  11
ccgggctggg agataatgtc agcatctcga gatgctgaca ttatctccca gctttttg      58


<210>  12
<211>  58
<212>  DNA
<213>  Artificial Sequence

<220>
<223>  ST3Gal1 shRNA, oligo sequence

<400>  12
ccgggcggga gaagaagccc aataactcga gttattgggc ttcttctccc gctttttg      58


<210>  13
<211>  21

<212>  DNA
<213>  Artificial Sequence

<220>
<223>  ST3Gal1 shRNA, target sequence

<400>  13
agagacttga gtggcgatta c                                                21


<210>  14
<211>  21
<212>  DNA
<213>  Artificial Sequence

<220>
<223>  ST3Gal1 shRNA, target sequence

<400>  14
gctgggagat aatgtcagca t                                                21


<210>  15
<211>  21
<212>  DNA
<213>  Artificial Sequence

<220>
<223>  ST3Gal1 shRNA, target sequence

<400>  15
gcgggagaag aagcccaata a                                                21

## Description

### BACKGROUND OF THE INVENTION

Glycosylation is an extremely important posttranslational modification of proteins. Aberrant glycosylation mainly affects the terminal portion of the carbohydrate moiety of glycoproteins and leads to the expression of tumor-associated antigens and the development of more advanced cancer staging and metastasis.

While cancer cells are now known to express tumor-associated antigens, they are often able to evade an immune response because of their ability to hide tumor antigens from the immune system and/or because the exposed tumor antigens are normal, nonmutated differentiation molecules or proteins which the human immune system normally recognizes or tolerates. This makes the elimination of cancer a challenging issue because, although cancerous cells proliferate in an uncontrolled manner, the cells do not necessarily appear to be "foreign" to the body and, therefore, are difficult to target.

To effectively treat a cancer, it would be preferred if the body's immune system could exhibit a positive immune response against these cancers. In addition, new and more effective anti-cancer therapeutic is crucial in the fight against cancer. Cell-based screening method is an important source of information in evaluating the efficacy and toxicity of new anti-cancer therapeutics, in early phases of preclinical drug development.

Accordingly, there is a need for a more effective cancer therapy via which a cancer cell is modified, rather than destroyed, to invoke an autologous or heterologous immune response to prevent further proliferation of cancer cells. What is further needed is a more effective screening method for potential cancer therapeutics. The present invention addresses these needs and other needs.

### BRIEF SUMMARY OF THE INVENTION

Embodiments of the present invention provide modified cancer cells, wherein the expression of ST3 beta-galactoside alpha-2,3-sialyltransferase 1 ("ST3Gal1", see SEQ ID NO:1) in the modified cancer cell is reduced compared to a conventional cancer cell. In an exemplary embodiment, at least one genetic construct is used to effectively reduce the expression of ST3Gal1 in the modified cancer cell.

According to one embodiment of the present invention, methods are provided for screening potential cancer therapeutics. The methods comprises (a) growing the modified cancer cell described herein in the presence of a potential cancer therapeutic and growing the modified cancer cell described herein in the absence of said potential cancer therapeutic; (b) determining the rate of growth of the modified cancer cell in the presence of the potential cancer therapeutic and the rate of growth or cell count of the modified cancer cell in the absence of the potential cancer therapeutic; and comparing the growth rate or cell count of modified cancer cell in the presence of the potential cancer therapeutic and in the absence of the potential cancer therapeutics, wherein a slower rate of growth or a lower cell count of the modified cancer cell in the presence of said therapeutic is indicative of a cancer therapeutic.

According to another embodiment of the present invention, methods for treating cancer or inhibiting cancer cell growth to a subject, comprising administering an effective amount of a genetic construct to suppress ST3Gal1 expression and an anti-cancer agent, wherein said effective amount of the genetic construct and the anti-cancer agent inhibit cancer cell growth in said subject, in a synergistic manner.

A further understanding of the nature and advantages of the present invention may be realized by reference to the remaining portions of the specification and the drawings.

### BRIEF DESCRIPTION OF THE DRAWINGS

FIG. 1A is a bar graph showing ST3Gal1 mRNA expression of the breast cancer cells transfected with scramble siRNA ("sc") or ST3Gal1 siRNA ("si", includes an antisense strand SEQ ID NO:2 and a sense strand SEQ ID: 3). FIG. 1B shows increased PNA-binding signals in breast cancer cells transfected with ST3Gal1 siRNA. FIG. 1C shows CD55 was identified as one of the PNA-binding proteins with increased signal (by 2.6 folds) after ST3Gal1 silencing of the breast cancer cells. Figure 1D shows increased CD55 binding signals after ST3Gal1 siRNA silencing in breast cancer cells.
FIG. 2A is a line graph illustrating breast cancer cells transfected with ST3Gal1 siRNA ("si") are more susceptible to anti-HLA antibody-mediated complement-dependent cytotoxicity (CDC) compared to breast cancer cells transfected with ST3Gal1 scrambled RNA("sc"). Figure 2B is a western blot image showing ST3Gal1 silencing affected the molecular weight of CD55.
FIG. 3 is a line graph illustrating increased C3 deposit on breast cancer cells transfected with ST3Gal1 siRNA (si) compared to those transfected with ST3Gal1 scrambled RNA (sc).
FIG. 4 is a bar graph illustrating breast cancer cells transfected with ST3Gal1 siRNA are more susceptible to anti-SSEA4 antibody mediated antibody-dependent cellular cytotoxicity (ADCC) compared to breast cancer cells transfected with ST3Gal1 scrambled RNA.
FIGS. 5 is a western blot image characterizing the sialylation of a CD55 molecule.
FIG. 6A is a direct LC-MS/MS analysis showing N-glycans of the CD55 molecules. FIG.6B shows the percentages of disialylated core 2 structure at m/z 864.426 (2+) and monosialylated core 2 structure at m/z 683.839 (2+) in breast cancer cells transfected with ST3Gal1 siRNA (si) and scrambled RNA (sc).
FIG. 7A and FIG. 7B are bar graphs illustrating the suppression of ST3Gal1 significantly reduced the proliferation of prostate cancer cells (FIG. 7A) and neuroblastoma cells (FIG. 7B) compared to conventional prostate cancer cells (transfected with scrambled RNA) and conventional neuroblastoma cells (transfected with scramble RNA).
FIG. 8A-FIG. 8C are bar graphs showing ST3Gal1 mRNA expression of the breast cancer cells transfected with scramble siRNA (sc) or various ST3Gal1 siRNAs and ST3Gal1 shRNAs.
FIG. 9 is a bar graph showing the synergistic effect of ST3Gal1 siRNA in combination with tamoxifen (Tam) and/or vandetanib (Van), compare to control group (ST3Gal1 scramble RNA in combination with tamoxifen and/or vandetanib)

### DETAILED DESCRIPTION OF THE INVENTION

As used herein, the articles "a" and "an" refer to one or more than one (*i.e*., at least one) of the grammatical object of the article. By way of example, "an element" means one element or more than one element.

An "effective amount," as used herein, refers to a dose of the genetic construct to suppress ST3Gal1 or that is sufficient to reduce the symptoms and signs of cancer, such as weight loss, pain and palpable mass, which is detectable, either clinically as a palpable mass or radiologically through various imaging means. The term "effective amount" and "therapeutically effective amount" are used interchangeably.

The term "subject" can refer to a vertebrate having cancer or to a vertebrate deemed to be in need of cancer treatment. Subjects include all warm-blooded animals, such as mammals, such as a primate, and, more preferably, a human. Non-human primates are subjects as well. The term subject includes domesticated animals, such as cats, dogs, etc., livestock (for example, cattle, horses, pigs, sheep, goats, etc.) and laboratory animals (for example, mouse, rabbit, rat, gerbil, guinea pig, etc.). Thus, veterinary uses and medical formulations are contemplated herein.

The terms "knock-down" and "silencing" are used interchangeably and refer to the reduced ST3Gal1 RNA or protein expression as compared to the RNA or protein expression prior to the introduction of the genetic construct.

### MODIFIED CANCER CELLS

The present invention provides modified cancer cell, wherein the expression of the ST3Gal1 protein and/or RNA in said modified cancer cells is reduced. In one embodiment, the modified cancer cell comprises about 1% to about 99% of ST3Gal1 glycosyltransferase, compared to a conventional cancer cell. In another embodiment, ST3Gal RNA and/or protein expression can in the modified cancer cell is reduced by 1-100 % compared to a conventional cancer cell. In yet another embodiment, ST3Gal1 protein or RNA expression in the conventional cancer cell is not altered by the genetic construct described herein or by any other means.

In one embodiment, the modified cancer cell is transfected with the genetic construct described herein to reduce intracellular ST3Gal1 glycosyltransferase or suppress ST3Gal1 RNA or protein expression. In other embodiment, the modified cancer cell maybe a solid or hematological cancer cell, benign or malignant (metastatic or nonmetastatic). Non limiting examples of the modified cancer cell are: breast, prostate, cervical, ovarian, endometrial, colon, liver, brain (e.g., neuroblastoma), pancreatic, bladder, esophagus, head and neck (e.g., nasopharyngeal cancer), renal, skin (e.g., melanoma or squamous cell carcinoma), gastric, testes, thyroid, lung, leukemia and lymphoma, and myelomas.

ST3Gal expression in a cancer cell can be reduced by 1 - 100% by administering a genetic construct into a cancer cell compared to a conventional cancer cell. For example, ST3Gal1 expression may be reduced by 10, 15, 20, 25, 30, 35, 40, 45, 50, 55, 60, 65, 70, 75, 80, 85, 90, 95, 99%, or any % or range of % therebetween in 1% increments (e.g., about 28%, about 10-40%, about 15-25%, etc). The RNA or protein expression can be measured by methods known in the arts, such as serial analysis of gene expression (SAGE), real time PCR or western blot.

In one exemplary embodiment, the modified cancer cell is a breast cancer cell deposited under ATCC Accession No. PTA-122610 (deposited with American Type Culture Collection, 10801 University Boulevard, Manassas, VA, 20110, USA on 20 October 2015)

### SCREENING OF POTENTIAL CANCER THERAPEUTICS

The invention provides methods of screening potential cancer therapeutics to identify suitable drugs for cancer treatment. The genetic construct described herein may be used for research purposes to inhibit the expression of ST3Gal1 RNA or protein in cells or experimental animals to appraise a potential cancer therapeutic. In one embodiment, the modified cancer cells described herein, which are transfected with a genetic construct to suppress the expression of ST3Gal1, are more susceptible to CDC and ADCC and can be used for screening potential cancer therapeutic, such as monoclonal antibody.

Thus, the present invention provides methods of screening potential cancer therapeutics comprising contacting such therapeutic with the modified cancer cells and measuring (i) the rate of growth of the modified cancer cells or (ii) the cell count of the modified cancer cells in the presence and in the absence of the potential cancer therapeutics being tested, by methods well known in the art, such as spectrophotometry, to determine if the therapeutics is capable of inhibiting the growth of cancer cells. In some embodiments, a slower rate of growth and/or a lower cell count of the modified cancer cell in the presence of the potential cancer therapeutic is indicative such therapeutic is effective against cancer cells.

### METHODS OF TREATING CANCER

According to the present invention, methods for inhibiting cancer cell growth are provided by administering an effective amount of (a) a genetic construct to inhibit or suppress ST3Gal1 protein and/or RNA expression and (b) an anti-cancer agent. Advantageously, this combination has synergistic effects on cancer cell inhibition, wherein one or even all of the lower dosages of the anti-cancer agents would not be sufficient to have a therapeutic effect when the respective anti-cancer agent is used in monotherapy.

CD55 molecule was identified as one of the target proteins of ST3Gal1. CD55 protects cells against complement attack by accelerating the decay of C3 and C5 to inhibit the activation of complement system and thereby preventing damage to cancer cells, from complement-mediated cytotoxicity.

Suppressing ST3Gal1 expression in the cancer cells is associated with reduced *O*-linked sialylation of the CD55 molecule and increased C3 deposit on cancer cell surface. The loss of CD55 function and increased C3 deposition enhance the susceptibility of cancer cells to CDC and ADCC and the efficacy of antibody-based immunotherapy.

In some embodiments, the genetic construct can be DNA or RNA or chimeric mixtures or derivatives or modified versions thereof, single-stranded or double-stranded. In other embodiment, the genetic construct is any RNA species such as but not limited to, single-stranded antisense oligonucleotides (ASO), small hairpin RNA (shRNA), small interfering RNA (siRNA, comprising short double-stranded RNA from about 17 nucleotides to about 29 nucleotides in length and a sense RNA strand and a complementary antisense RNA strand annealed together by standard Watson-Crick base-pairing interactions), microRNA (miRNA), endoribonuclease-prepared siRNA (esiRNA), natural antisense short interfering RNA (natsiRNA), RNA interference (RNAi), which can involve the use of siRNA, to knockdown the expression of specific genes in plants and several vertebrate species including the mouse and zebrafish. See, for example, US Pat. No. 7,416,849.

The genetic construct may further comprises a base moiety, sugar moiety, or phosphate backbone, for example, to improve stability of the molecule, hybridization, etc. The genetic construct may include other appended groups such as peptides (e.g., for targeting host cell receptors), or agents facilitating transport across the cell membrane (see, e.g., Letsinger et al., 1989, Proc. Natl. Acad. Sci. U.S.A. 86:6553-6556).

In some embodiments, the ASO comprises a nucleotide sequence at least 90, 91, 92, 93, 94, 95, 96, 97, 98, 99, or 100% homologous to or substantially identical to a sequence selected from SEQ ID NO: 2, 4, 6, and 8. The ASO must survive and bind to the complementary target sequence of ST3Gal1 mRNA to prevent translation alone, as a single strand.

In some embodiments, the siRNA comprises a sense strand nucleotide sequence at least 90, 91, 92, 93, 94, 95, 96, 97, 98, 99 or 100% homologous or substantially identical to a sequence selected from SEQ ID NOs:3, 5, 7 and 9, and an antisense strand nucleotide sequence at least 90, 91, 92, 93, 94, 95, 96, 97, 98, 99,or 100% homologous or substantially identical to a sequence selected from SEQ ID NO: 2, 4, 6, and 8.

In some embodiments, the shRNA has a nucleotide sequence at least 90, 91, 92, 93, 94, 95, 96, 97, 98, 99 or 100% homologous or substantially identical to a sequence selected from SEQ ID NOs: 10, 11 and 12.

Antisense approaches involve the design of a genetic construct that are complementary to a target sequence of ST3Gal1 mRNA, such as SEQ ID NOs:13, 14 or 15. The target region of SEQ ID NO: 13 is 3UTR whereas the target region of SEQ ID Nos: 14 and 15 is CDS. Absolute complementarity, although preferred, is not required. A sequence "complementary" to a ST3Gal1 target sequence, as referred to herein, means a sequence having sufficient complementarity to be able to hybridize with the target RNA sequence, forming a stable duplex; in the case of double-stranded antisense nucleic acids, a single strand of the duplex DNA may thus be tested, or triplex formation may be assayed. The ability to hybridize will depend on both the degree of complementarity and the length ofthe antisense nucleic acid. Generally, the longer the hybridizing RNA, the more base mismatches with the target RNA it may contain and still form a stable duplex (or triplex, as the case may be). One skilled in the art can ascertain a tolerable degree of mismatch by use of standard procedures to determine the melting point of the hybridized complex.

In determining the degree of "complementarity" between the genetic construct and the ST3Gal1 target sequence disclosed herein (SEQ ID Nos: 13, 14 or 15), the degree of complementarity is expressed as the percentage identity between the genetic construct and the reverse complement of the ST3Gal1 target sequence that best aligns therewith. The percentage is calculated by counting the number of aligned bases that are identical as between the 2 sequences, dividing by the total number of the genetic construct, and multiplying by 100. Polynucleotide alignments, percentage sequence identity, and degree of complementarity may be determined for purposes of the disclosure using the ClustalW algorithm using standard settings.

In some embodiments, the genetic construct includes 1, 2, 3, or 4 (or more) mismatches as compared to the best-aligned target sequence of ST3Gal1, and still sufficiently binds to the target sequence to effect inhibition of ST3Gal1 mRNA or protein expression. The destabilizing effect of mismatches on Watson-Crick hydrogen-bonded duplex may, for example, be compensated by increased length of the genetic construct.

Regardless of the choice of ST3Gal1 target sequence, an in vitro study can be performed by a person skilled in the art to compare levels of ST3Gal1 RNA or protein expression of the tested genetic construct complementary to ST3Gal target sequence with that of a control oligonucleotide (e.g., a scramble RNA). It is preferred that the control oligonucleotide is of approximately the same length as the tested genetic construct.

Vectors to introduce the genetic construct for recombination and for extrachromosomal maintenance are known in the art, and any suitable vector may be used. Methods for introducing the genetic construct into cells, such as viral transfection are known in the art, and the choice of method is within the competence of a skilled artisan.

Some embodiments of the present invention utilize vectors that can be delivered to the cancer cells. As used herein, the term "vector" refers to any viral or non-viral vector, as well as any plasmid, cosmid, phage or binary vector in double or single stranded linear or circular form that may or may not be self-transmissible or mobilizable, and that can transform prokaryotic or eukaryotic host cells either by integration into the cellular genome or which can exist extrachromosomally (e.g., autonomous replicating plasmid with an origin of replication). Any vector known in the art is envisioned for use in the practice of this invention. Non limiting examples of viral vector include adeno-associated viral vector, lentivirus vector, adenoviral vector, polioviral vector, herpes simplex viral vector, or murine-based viral vector. Non limiting examples of non-viral vector include hydrodynamic or direct injection, cholesterol conjugation, liposomes and lipoplexes, polymers and peptide delivery systems or surface modified LPD nanoparticles (K. Gao et al., Non viral Methods for siRNA Delivery, Mol Pharm. 2009 Jun 1; 6(3): 651-658).

The genetic construct to suppress ST3Gal1 expression and the anti-cancer agent can be administered at any effective amount. In some embodiments, they may be administered at a dose ranging from about 0.01 µg to about 5 g or from about 0.1 µg/kg bodyweight to about 200 mg/kg bodyweight. The dosage of the genetic construct to suppress ST3Gal1 expression or the anti-cancer agent will depend on the severity of the condition being treated, the particular formulation, and other clinical factors such as weight and the general condition of the recipient and route of administration.

Useful dosages of the genetic construct to suppress ST3Gal1 expression and the anti-cancer agent are determined by comparing their *in vitro* activity, and *in vivo* activity in animal models. Methods for the extrapolation of effective dosages in mice, and other animals, to humans are known in the art; for example, see U.S. Pat. No. 4,938,949.

In accordance with the methods provided herein, the genetic construct to suppress ST3Gal1 expression and the anti-cancer agent can be delivered by a variety of routes including, but not limited to, injection (e.g., subcutaneous, intramuscular, intravenous, intra-arterial, intraperitoneal, intradermal); cutaneous; dermal; transdermal; oral ( e.g., tablet, pill, liquid medicine, edible film strip); implanted osmotic pumps; suppository, aerosol spray, topical, intra-articular, ocular, nasal inhalation, pulmonary inhalation, impression into skin, vaginal, or provided within an implant such as an osmotic pump in a graft comprising appropriately transformed cells.

The combination of the genetic construct and the anti-cancer agent may be administered in a single dose treatment or in multiple dose treatments, over a period of time appropriate to the condition being treated. The genetic construct to suppress ST3Gal1 expression and the anti-cancer agent may conveniently be administered at appropriate intervals, for example, once a day, twice a day, three times a day, once every second day, once every three days or once every week, over a period of at least 3 months or until the symptoms and signs of the condition resolved.

Non-limiting examples of anti-cancer agents can be found in Cancer Principles and Practice of Oncology by V. T. Devita, T. Lawrence and S. Rosenberg (editors), 9th edition (2011), Lippincott Williams & Wilkins Publishers. A person of ordinary skill in the art would be able to discern which combinations of anti-cancer agent would be useful based on the particular characteristics of the drugs and the cancer involved. Non limiting examples of such anti-cancer agents include: chemotherapy (e.g., alkylating agents, platinum analogs, anti-metabolites, targeted therapy such as estrogen receptor (ER) inhibitor and RET (REarranged during Transfection) inhibitor), surgery, radiotherapy, biotherapeutics (e.g., anti-angiogenesis agent, interleukin therapy, gene therapy, cancer vaccine, antibody, immunotherapy), or a combination thereof. In an exemplary embodiment, the antibody is against a tumor associate carbohydrate antigen selected from Globo H, SSEA-3, SSEA-4, Gb4 or Bb4.

In one embodiment, the anti-cancer agent is a cancer vaccine, an antibody, an estrogen receptor (ER) inhibitor, an RET (REarranged during Transfection) inhibitor or a combination thereof. Non limiting examples of ER inhibitor are tamoxifen and bazedoxifene. Non limiting examples of RET inhibitor are: Cabozantinib, Vandetanib, alectinib, lenvatinib, nintedanib Ponatinib, Sunitinib, and Sorafenib.

Embodiments of the present invention are illustrated by the following examples, which are not to be construed in any way as imposing limitations upon the scope thereof. During the studies described in the following examples, conventional procedures were followed, unless otherwise stated.

### Example 1: The Identification of ST3Gal1 targeted-proteins

An *in vitro* study was conducted to identify ST3Gal1 targeted proteins by comparing PNA binding proteins between ST3Gal1-silenced cells and control cells.

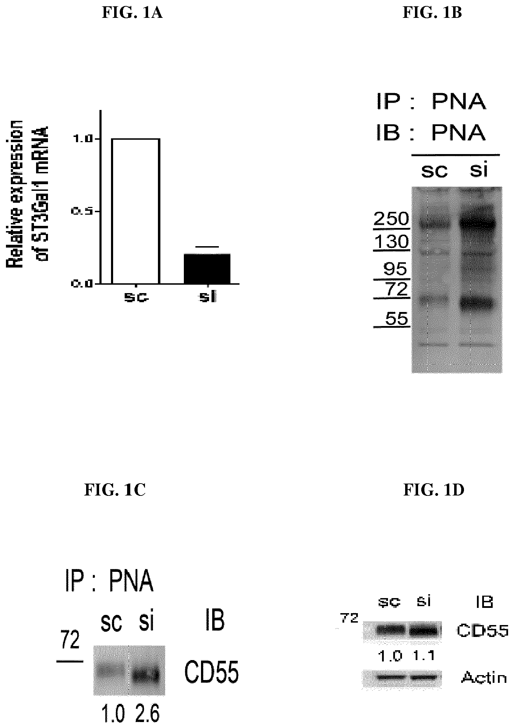

MDA-MB-231 breast cancer cells (available from Bioresource Collection and Research Center, Taiwan) were transfected with scramble siRNA (sc) or ST3Gal1 (si) siRNA with an antisense strand (SEQ ID NO:2) and a sense strand (SEQ ID NO:3). The expression levels of the ST3Gal1 mRNA were analyzed by qPCR analysis and normalized to GAPDH mRNA levels. As shown in Figure 1A, the expression of ST3Gal1 RNA was significantly reduced by ST3Gal1siRNA to 28.5 ± 8.2 % of that of sc transfected breast cancer cells.

To examine the protein target of ST3Gal1 in breast cancer cells, total cell lysates from scramble or ST3Gal1 siRNA treated breast cancer cells were prepared for PNA lectin blot analysis after precipitation with agarose bound PNA. ST3Gal1 catalyzes the sialylation of galactosyl (β-1, 3) N-acetylgalactosamine, by adding sialic acid to Core 1 O-glycans. Since Peanut agglutinin (PNA) binds preferentially to Core 1 O-glycans (without the added sialic acid), ST3Gal1 knock down is expected to increase PNA binding to Core 1 O-glycans.

As expected, PNA-binding signals in breast cancer cells increased at about 72, 130 and 250 kDa molecular weight, after ST3Gal1 knock down (Figure 1B). Using in-gel digestion and LTQ-FTD MASS spectrometry, CD55 was identified as one of the PNA-binding proteins with increased signal (by 2.6 folds) after ST3Gal1 knock down and the molecular weight of CD55 is slightly lower in ST3Gal1 silenced cells (Figure 1C). However, the total CD55 protein levels remained similar (sc vs. si: 1.0 vs.1.1) (Figure 1D).

### Example 2: ST3Gal1 silencing sensitizes breast cancer cells to complement-dependent cytotoxicity

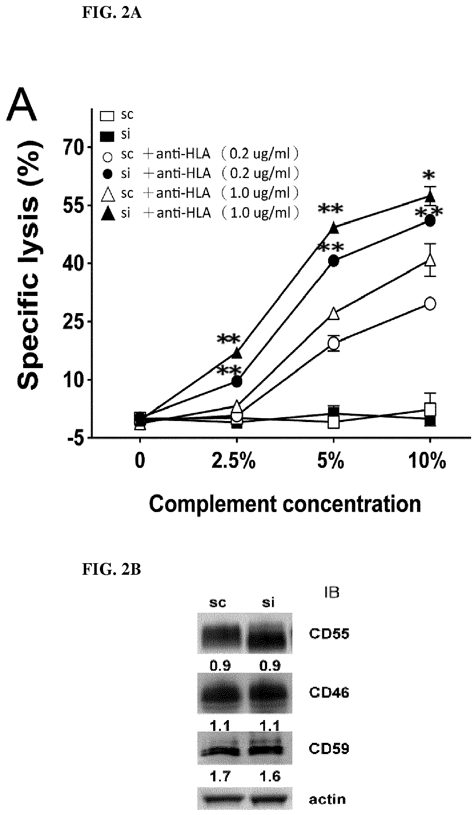

CD55 the cell surface regulates the complement system by accelerating the disassembly of C3bBb complex, interrupting the complement cascade, and preventing complement-dependent cytotoxicity (CDC). An *in vitro* experiment was conducted to determine if altered ST3Gal1-mediated *O*-linked sialylation affect the function of CD55. Fig 2A shows the effect of scramble (sc) or ST3Gal1 siRNA (si) in breast cancer cells, in the presence of anti-HLA antibodies (0.2 µg/ml or 1 µg/ml).

As shown in Figure 2A, ST3Gal1 knock down enhances cell death in the presence of antibody and/or complement, in a dose-dependent manner.

Specifically, the % of ST3Gal1 silenced breast cancer cell lysis, in the presence of 0.2 µg/ml anti-HLA antibodies and 10 % rabbit complement ranged from 29.6 ± 0.9 to 51.1 ± 2.2 %. The % of lysis of ST3Gal1 silenced breast cancer cells in the presence of 1 µg/ml anti-HLA antibodies and 10 % rabbit complement ranged from 40.9 ± 5.9 to 57.4 ± 4.2 % (Figure 2A). Breast cancer cells were more susceptible to CDC attack if ST3Gal1 is silenced and the surface CD55 is de-sialylated.

Figure 2B shows ST3Gal1 siRNA only modified the molecular weight of CD55, not the molecular weight other CDC regulatory proteins, such as CD46 and CD59. In addition, the expression levels of CD46 or CD59 were unaffected in breast cancer cells transfected with ST3Gal1 siRNA.

### Example 3: ST3Gal1 silencing sensitizes breast cancer cells to CDC by increased C3 deposition

An *in vitro* evaluation was performed to assess the decay-accelerating ability of ST3Gal1 silenced breast cancer cells with de-sialylated CD55, by measuring the deposition of C3 fragments on the cancer cell surface.

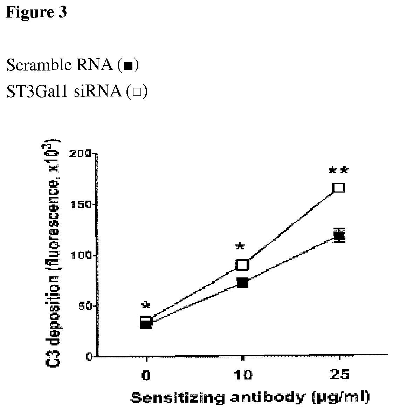

Breast cancer cells were incubated under non-lytic conditions with human serum and deposited C3 were detected by flow cytometric analysis using anti-C3 antibody. As shown in Figure 3, C3 deposit on the cancer cell surface is correlated with the concentration of anti-human leukocyte antigens (HLA) antibody. There is minimal C3 deposit in both scramble RNA (sc) and ST3Gal1 siRNA (si) transfected breast cancer cells in the absence ofanti-HLA antibody (geometric mean fluorescence: sc, 31.5 ± 0.2 x 10³; si, 35.2 ± 0.6 x 10³). C3 deposit on the surface of si transfected breast cancer cells was significantly higher than that of sc transfected breast cancer cells at 10 µg/ml and 25 µg/ml of antibody (genomic median fluorescence: 72.1 ± 4.8 x 10³ vs. 89.5 ± 5.5 x 10³ for sc vs. si, respectively, at 10 µg/ml antibody and 118.0 ± 6.4 x 10³ vs. 164.8 ± 0.8 x 10³ for sc vs. si, respectively, at 25 µg/ml antibody). The result suggests ST3Gal1 silencing increases C3 deposits and reduces decay-accelerating activity of surface-expressed CD55.

### Example 4: ST3Gal1 silencing sensitizes breast cancer cells to antibody-dependent cell-mediated (ADCC) cytotoxicity

The interaction of de-sialylated CD55 on the surface of ST3Gal1 silenced breast cancer cells and peripheral blood mononuclear cells (PBMC)-mediated ADCC was investigated

MDA-MB-231 breast cancer cells were transfected with scramble RNA (sc) or ST3Gal1 siRNA (si) according to Example 1. After four days, cells were collected and analyzed for the expression of membrane complement regulatory proteins (mCRPs) and target antigen by fluorescence-activated cell sorting (FACS). There was no change in the expression levels of mCRPs (CD46, CD55, and CD59) and target antigens (HLA and SSEA4) with ST3Gal1 silencing.

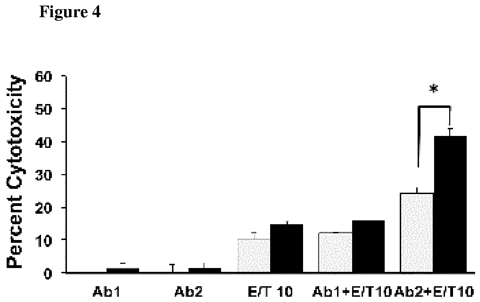

ADCC assay was performed using human PBMC cells as effectors, anti-SSEA4 as antibody, and ST3Gal1siRNA transfected MDA-MB-231 cells as targets. Figure 4 shows ST3Gal1 silenced breast cancer cells were significantly more sensitive to ADCC than scramble siRNA transfected breast cancer cells. The mean percentages of lysis are 24.2 ± 1.7 % and 41.5 ± 2.4 % for sc and si, respectively at Effector:Target ratio of 10:1 ratio (E/T10), and the difference is statistically significant. IgG3 isotype control (Ab1) and anti-SSEA4 antibody (Ab2) alone had minimal cell lysis in the absence of PMBCs.

### Example 5: Glycosylation pattern of CD55 in ST3Gal1 silenced breast cancer cells

The glycosylation pattern of CD55 was analyzed, using breast cancer cells transfected with ST3Gal1 siRNA and lysed with RIPA buffer. The transfected and lysed breast cancer cells were treated with deglycosylation enzymes (PNGsse F, O-glycosidase, and neuraminidase) and analyzed using western blot. The calculated molecular weight of CD55 is about 41 kDa. CD55 in sc transfected (control) breast cancer cells migrated as a 70 kDa protein, whereas CD55 in ST3Gal1 silenced breast cancer cells migrated faster with a lower molecular weight (Figure 1C). After the removal of *N*-glycans by PNGase F, the molecular weight of CD55 in sc transfected breast cancer cells reduced slightly. However, the molecular weight of CD55 in ST3Gal1 silenced breast cancer cells is still lower than that of CD55 in sc treated breast cancer cells. The result shows after PNGase F treatment, the CD55 molecular weights of sc transfected and ST3Gal1 silenced breast cancer cells are different, indicating ST3Gal1 mediates sialylation of *O-*linked glycans on CD55.

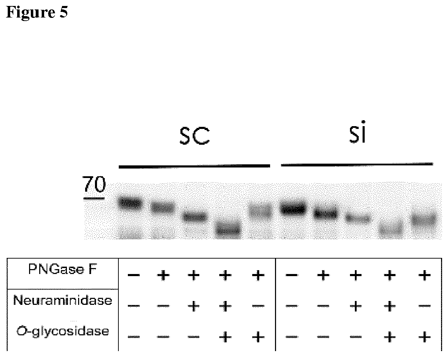

The sialic residues of the CD55 molecules were removed using neuraminidase, which catalyzes the hydrolysis of α2-3, α2-6, and α2-8 linked *sialic* acid residues from glycoproteins and oligosaccharides. Treatment of CD55 molecules with neuraminidase reduced the molecular weight of CD55 in both ST3Gal1 silenced and sc transfected (control) breast cancer cells (Figure 5). Double digestion of CD55 molecules with PNGase F and neuraminidase further reduced the molecular weight of CD55. These findings suggest that sialylation of CD55 is *O*-linked.

To further confirm this finding, sc transfected and ST3Gal1 siRNA transfected breast cancer cells were treated with *O*-Glycosidase to catalyze the removal of Core 1 and Core 3 *O-*linked disaccharides from glycoproteins. The molecular weight CD55 molecule was reduced in both sc transfected and ST3Gal1 silenced breast cancer cells, but the molecular weight of CD55 is lower in ST3Gal1 siRNA transfected breast cancer cells compare to that of the sc transfected breast cancer cells. Triple digestion of CD55 molecules with PNGase F, neuraminidase, and *O-*glycosidase reduced the molecular weight of CD55 molecule to ∼50KD in both ST3Gal1 silenced and sc transfected breast cancer cells, as shown in FIG. 5. These findings confirmed that CD55 is an *O*-linked sialylated glycoprotein, catalyzed by ST3Gal1.

### Example 6: N- and O-glycosylation profiles of CD55

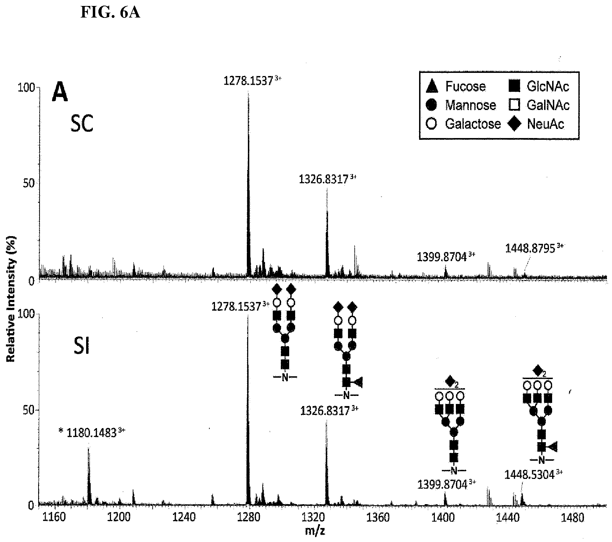

A direct LC-MS/MS analysis showed the majority of N-glycans of the CD55 molecules, in ST3Gal1 silenced and sc transfected breast cancer cells, comprised complex bi- and tri-antennary structures bearing terminal sialic acids, with and without core fucosylation. The bi- and tri-antennary structures were all detected as glycoforms of the expected tryptic peptide GSQWSDIEEFCNR, previously reported as the single *N*-linked glycosylation site. The relative amount of bi- and tri-antennary structures did not change significantly upon ST3Gal1 silencing (Figure 6A). The same analysis of the CD55 molecules in ST3Gal1 silenced and sc transfected breast cancer cells failed to identify any of the possible 24 predicted *O*-linked glycosylation sites located at serine/threonine-rich stalk region (www.cbs.dtu.dk/services/netogly). Instead, the LC-MS/MS analysis of the released, permethylated *O*-glycans showed the major structure of CD55 molecule was based on core 1, with and without sialic acid, and core 2 with up to 2 sialic acids. The sialylation of core 1 and core 2 O-glycan on CD55 regulates the activity of CD55.

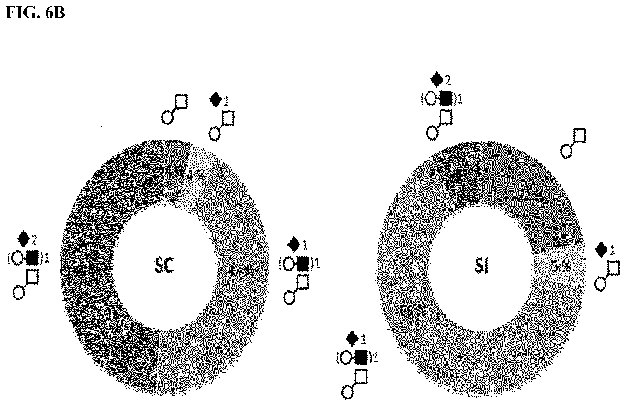

The relative amount of disialylated core 2 structure at m/z 864.426 (2+) was significantly reduced, whereas the relative amount of monosialylated core 2 structure at m/z 683.839 (2+) increased in ST3Gal1 silenced breast cancer cells (Figure 6B). The LC-MS data suggests ST3Gal1 plays an important role in 2-3-sialylation of *O*-glycans but not *N*-glycans of the CD55 molecule. Impairment of 2-3-sialylation ofthe *O*-glycans ofthe CD55 molecule increases non-sialylated core 1 and monosialylated core 2, at the expenses of disialylated core 2 structure. On the other hand, the relative amount of the monosialylated core 1 *O*-glycan of the CD55 molecule (m/z 895.462) was similar in ST3Gal1 silenced and sc transfected breast cancer cells, probably due to compensation of 2-6-sialylation.

### Example 7: The Effect of ST3Gal1 knockdown on the proliferation of prostate cancer cells and neuroblastoma cells.

Prostate cancer cells (PC3, Bioresource Collection and Research Center, Taiwan) and neuroblastoma cells (SH-SY5Y, obtained from Professor Yi-Cheng Chen, Mackay Medical College, Taiwan) were transfected with a scramble RNA (sc) or ST3Gal1 siRNA (si) according to Example 1. The cell proliferation was determined by AlamarBlue assay using Victor X3 plate reader to measure the fluorescence (590 nm) that was used to present the level of cell proliferation.

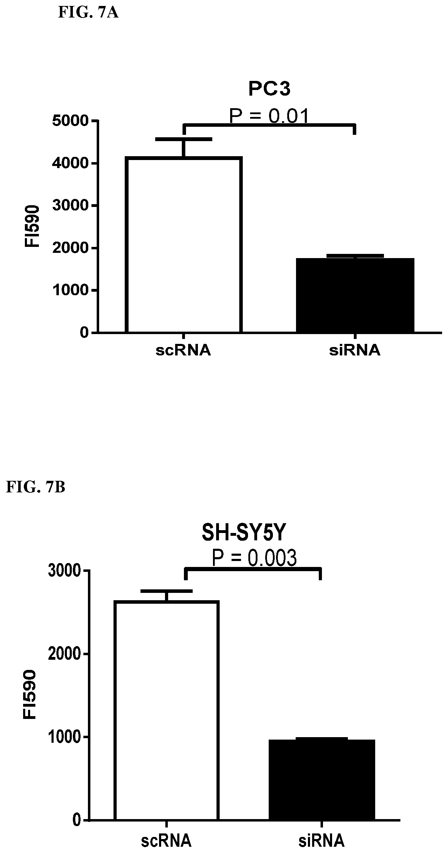

FIG. 7A and FIG. 7B show that the suppression of ST3Gal1 by siRNA (siRNA) significantly reduced the proliferation of prostate cancer cells and neuroblastoma cells compared to no ST3Gal1 suppression (sc RNA).

### Example 8: The Effect of siRNAs and shRNAs on ST3Gal1 Suppression in Breast Cancer Cells.

MDA-MB-231 breast cancer cells were transfected with scramble siRNA (sc), a vector without shRNA(pVoid), shRNA1-3 (SEQ ID NO:11), shRNA 1-4(SEQ ID NO:12), or the siRNA listed in Table 1 below:

| siRNA | Antisense strand | Sense strand |
|---|---|---|
| siRNA-1 | AUCUCAGGCCCAUAAGAAGACUCCC (SEQ ID NO:4) | GGGAGUCUUCUUAUGGGCCUGAGAU (SEQ ID NO:5) |
| siRNA-2 | UCAUGCUGACAUUAUCUCCCAGCUC (SEQ ID NO:6) | GAGCUGGGAGAUAAUGUCAGCAUGA (SEQ ID NO:7) |
| siRNA-3 | UUGAGAAGAUGACCGAGAGGAUGCC (SEQ ID NO:8) | GGCAUCCUCUCGGUCAUCUUCUCAA (SEQ ID NO:9) |
| siRNA-A | commercially available as catalog sc-37007 St3Gal-I siRNA from Santa Cruz Biotechnology, USA, nucleotide sequence not available | |
| siRNA-7 | commercially available as catalog sc- 106571 St3Gal-I siRNA from Santa Cruz Biotechnology, USA, nucleotide sequence not available | |

The expression levels of the ST3Gal1 mRNA were analyzed by qPCR analysis and normalized to GAPDH mRNA levels.

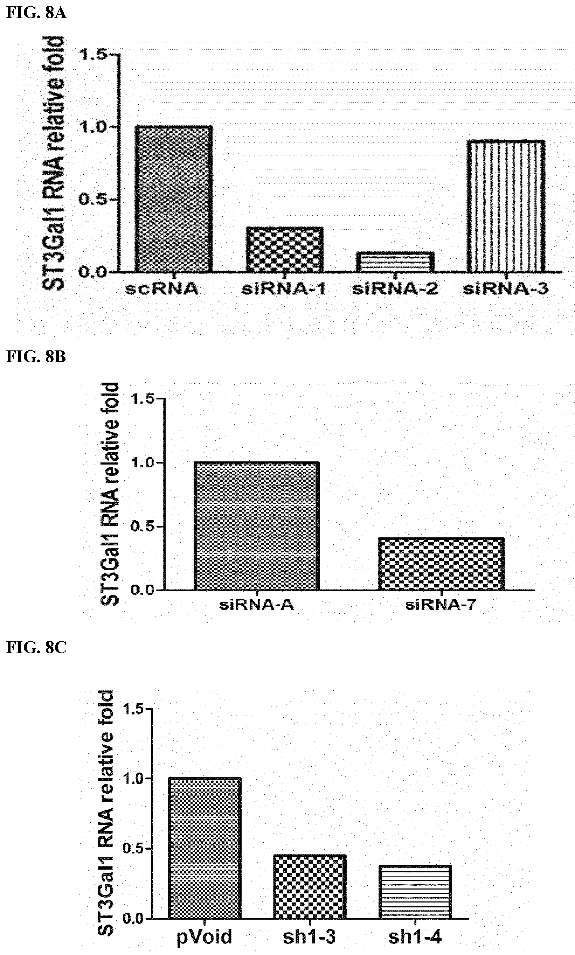

As shown in Figure 8A-FIG. 8C, the expression of ST3Gal1 RNA was reduced in siRNA-1, siRNA-2, siRNA-3, siRNA-7, shRNA1-3, and shRNA1-4 transfected breast cancer cells compared to sc RNA, siRNA-A or pVoid transfected breast cancer cells.

### Example 8: The Synergistic Effect of ST3Gal1 siRNAs in combination with anti-cancer agent in Breast Cancer Cells.

An *in vitro* evaluation of the effect of ST3Gal1 siRNA (to knock down ST3Gal1 expression) and anti-cancer agent was performed using breast cancer cells.

MCF7 breast cancer cells were treated with one of the regimes in Table 2 and cell growth was monistored by xCELLigence System (ACEA Biosciences, Inc., USA)

**Table 2**

| Control Group | Treatment Group |
|---|---|
| Scramble RNA + DMSO | ST3Gal1 siRNA + DMSO |
| Scramble RNA + tamoxifen | ST3Gal1 siRNA + tamoxifen |
| Scramble RNA+ vandetanib | ST3Gal1 siRNA+ vandetanib |
| Scramble RNA+ vandetanib + tamoxifen | ST3Gal1 siRNA+ vandetanib + tamoxifen |

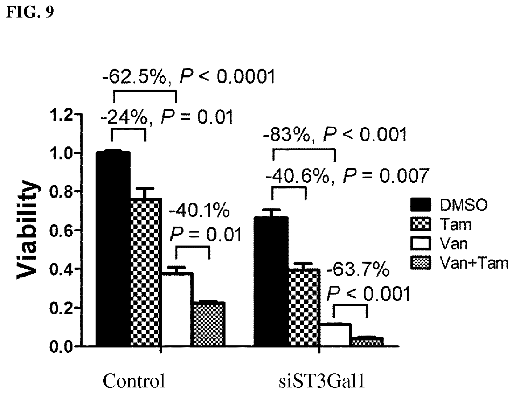

FIG. 9 shows the synergistic effect of ST3Gal1 knock down and an anti-cancer agent, such as tamoxifen, vandetanib or combination thereof. Cancer cell viability for tamoxifen alone was 76%, for vandetanib alone was 37.5%, for tamoxifen and vandetanib combination was 22% for ST3Gal1 siRNA alone was 65%, for ST3Gal1 siRNA and tamoxifen combination was 40%, or ST3Gal1 siRNA and vandetanib combination was 17%, for ST3Gal1 siRNA, tamoxifen, and vandetanib combination was 5%.

## Claims

1. A modified cancer cell, wherein the expression of ST3Gal1 glycosyltransferase (ST3Gal1) in the modified cancer cell is reduced.

2. The modified cancer cell of claim 1, wherein said expression of ST3Gal1 in the modified cancer cell is reduced by 1-99% compared to a conventional cancer cell.

3. The modified cancer cell of claim 1, wherein the modified cancer cell is a modified breast cancer cell, a modified prostate cancer cell or a modified neuroblastoma cell.

4. The modified breast cancer cell of claim 3, having an ATCC deposit number PTA-122610.

5. An *in vitro* method for screening a potential cancer therapeutic, comprises:
(a) growing the modified cancer cell of claim 1 in the presence of the potential cancer therapeutic and growing the modified cancer cell of claim 1 in the absence of said potential cancer therapeutic;
(b) determining the rate of growth or cell count of the modified cancer cell in the presence of the potential cancer therapeutic and the rate of growth or cell count of the modified cancer cell in the absence of the potential cancer therapeutic; and
(c) comparing the growth rate or cell count of the modified cancer cell in the presence of the potential cancer therapeutic and in the absence of the potential cancer therapeutic, wherein a slower rate of growth or a lower cell count of the modified cancer cell in the presence of said therapeutic is indicative of a cancer therapeutic.

6. The method of claim 5, wherein the potential cancer therapeutic is a monoclonal antibody.

7. A genetic construct that suppresses ST3Gal1 expression for use in treating cancer or inhibiting cancer cell growth in a subject, wherein said genetic construct is to be administered in combination with an anti-cancer agent.

8. The genetic construct for use according to claim 7, wherein the genetic construct for suppressing ST3Gal1 expression is selected from a single strand antisense oligonucleotide, a small interfering RNA (siRNA) and a small hairpin RNA (shRNA)

9. The genetic construct for use according to claim 8, wherein the single strand antisense oligonucleotide is at least 90% homologous to a nucleotide sequence selected from SEQ ID NOs:2, 4, 6, and 8.

10. The genetic construct for use according to claim 8, wherein the siRNA comprises an antisense strand at least 90% homologous to a nucleotide sequence selected from SEQ ID NOs:2, 4, 6, and 8 and a sense strand at least 90% homologous to a nucleotide sequence selected from SEQ ID NOs:3, 5, 7 and 9.

11. The genetic construct for use according to claim 8, wherein the shRNA comprises a nucleotide sequence at least 90% homologous to a sequence selected from SEQ ID NOs: 10, 11 and 12.

12. The genetic construct for use according to claim 7, wherein the genetic construct comprises a nucleotide sequence at least 90% complementary to a sequence selected from SEQ ID NOs: 13, 14 and 15.

13. The genetic construct for use according to any of claims 7 to 12, wherein the genetic construct is to be administered with an anti-cancer agent that is a cancer vaccine, an antibody, an estrogen receptor (ER) inhibitor, which is preferably tamoxifen, a rearranged during transfection (RET) inhibitor, which is preferably vandetanib, or a combination thereof.

14. The genetic construct for use according to claim 13, wherein the antibody is against a tumor associate carbohydrate antigen selected from Globo H, SSEA-3, SSEA-4, Gb4 or Bb4.
